# EUROPEAN PATENT APPLICATION

(11) **EP 2 039 362 A1**
(43) Date of publication of application: **25.03.2009**
(21) Application number: 07767674.0
(22) Date of filing: 27.06.2007
(51) Int. Cl.: A61K 31/736, A61P 1/02

(54) **TOOTH ENAMEL DISSOLUTION INHIBITOR**

(30) Priority: 28.06.2006 JP 2006178817
(71) Applicant: Otsuka Pharmaceutical Co., Ltd., Tokyo 101-8535 (JP)
(72) Inventor: SHINOHARA, Shigeo, Otsu-shi Shiga 520-0002 (JP); IGARASHI, Sachiyo, Otsu-shi Shiga 520-0002 (JP); TAKASU, Osamu, Otsu-shi Shiga 520-0002 (JP)
(74) Representative: HOFFMANN EITLE
(86) International application number: PCT/JP2007/062871
(87) International publication number: WO 2008/001800

(57) **Abstract**

An object of the present invention is to provide a tooth enamel dissolution (decalcification) inhibitor that can inhibit the dissolution of tooth enamel to keep teeth healthy.

The tooth enamel dissolution inhibitor contains at least one member selected from the group consisting of guar gum, glucomannan, β-cyclodextrin, tragacanth gum, carrageenan, locust bean gum, hyaluronic acid, pullulan, pectin, and xanthan gum.

## Description

### TECHNICAL FIELD

The present invention relates to a tooth enamel dissolution (decalcification) inhibitor used to inhibit dissolution (decalcification) of tooth enamel.

### BACKGROUND ART

Against the backdrop of richer eating habits in recent years, various foods have been developed in the pursuit of greater functionality and palatability, while more and more foods tend to cause the dissolution of tooth enamel.

For example, the present inventors have previously discovered that acids, such as citric acid, malic acid, ascorbic acid, etc., stimulate the secretion of human β defensin i.e., antimicrobial peptide. Promoting human β defensin secretion in the oral cavity using these acids is reportedly effective in preventing or treating dental caries, periodontitis, and halitosis (see Patent Document 1). On the other hand, there is concern that the application of acid to the oral cavity may cause the dissolution (decalcification) of the enamel that forms teeth.

Considering such known techniques, there is a demand for the development of effective enamel dissolution inhibitors to maintain the teeth in a healthy condition and to keep the oral cavity healthy by inhibiting the dissolution of tooth enamel.
Patent Document 1: WO 2005/027893

### DISCLOSURE OF THE INVENTION

### PROBLEMS TO BE SOLVED BY THE INVENTION

The present invention aims to provide a tooth enamel dissolution (decalcification) inhibitor that inhibits the dissolution of tooth enamel to keep teeth healthy.

### MEANS FOR SOLVING THE PROBLEM

The present inventors carried out extensive research to solve the above-described problems and found that guar gum, glucomannan, β-cyclodextrin, tragacanth gum, carrageenan, locust bean gum, hyaluronic acid, pullulan, pectin, and/or xanthan gum are effective for inhibiting the dissolution (decalcification) of enamel. Particularly, the inventors found that these components exhibit an effective inhibitory activity on the dissolution of tooth enamel caused by citric acid, malic acid, or ascorbic acid. The present invention has been accomplished as a result of further studies based on these findings.

Specifically, the present invention is provided in the following embodiments:
Item 1. A tooth enamel dissolution inhibitor comprising at least one member selected from the group consisting of guar gum, glucomannan, β-cyclodextrin, tragacanth gum, carrageenan, locust bean gum, hyaluronic acid, pullulan, pectin, and xanthan gum. Item 2. The dissolution inhibitor according to Item 1 comprising guar gum and/or glucomannan.
Item 3. The dissolution inhibitor according to Item 1, which is used for inhibiting tooth enamel dissolution caused by acid.
Item 4. A food for inhibiting tooth enamel dissolution comprising at least one member selected from the group consisting of guar gum, glucomannan, β-cyclodextrin, tragacanth gum, carrageenan, locust bean gum, hyaluronic acid, pullulan, pectin, and xanthan gum.
Item 5. The food for inhibiting tooth enamel dissolution according to Item 4 comprising guar gum and/or glucomannan.
Item 6. A medicine for inhibiting tooth enamel dissolution comprising at least one member selected from the group consisting of guar gum, glucomannan, β-cyclodextrin, tragacanth gum, carrageenan, locust bean gum, hyaluronic acid, pullulan, pectin, and xanthan gum.
Item 7. The medicine for inhibiting tooth enamel dissolution according to Item 6 comprising guar gum and/or glucomannan.
Item 8. The medicine for inhibiting tooth enamel dissolution according to Item 6, which is used for inhibiting tooth enamel dissolution caused by acid.
Item 9. An oral cavity product for inhibiting tooth enamel dissolution comprising at least one member selected from the group consisting of guar gum, glucomannan, β-cyclodextrin, tragacanth gum, carrageenan, locust bean gum, hyaluronic acid, pullulan, pectin, and xanthan gum.
Item 10. The oral cavity product for inhibiting tooth enamel dissolution according to Item 9 comprising guar gum and/or glucomannan.
Item 11. The oral cavity product for inhibiting tooth enamel dissolution according to Item 9, which is used for inhibiting tooth enamel dissolution caused by acid.
Item 12. A method for inhibiting tooth enamel dissolution comprising applying an effective amount of at least one member selected from the group consisting of guar gum, glucomannan, β-cyclodextrin, tragacanth gum, carrageenan, locust bean gum, hyaluronic acid, pullulan, pectin, and xanthan gum, to an oral cavity.
Item 13. The method according to Item 12 applying an effective amount of guar gum and/or glucomannan to an oral cavity.
Item 14. The method according to claim 12, which is a method for inhibiting tooth enamel dissolution caused by acid.
Item 15. Use of at least one member selected from the group consisting of guar gum, glucomannan, β-cyclodextrin, tragacanth gum, carrageenan, locust bean gum, hyaluronic acid, pullulan, pectin, and xanthan gum, for production of a tooth enamel dissolution inhibitor.
Item 16. Use of guar gum and/or glucomannan for production of a tooth enamel dissolution inhibitor.
Item 17. The use according to Item 15 or 16, wherein the tooth enamel dissolution inhibitor is used for inhibiting tooth enamel dissolution caused by acid.

### Effect of the Invention

The tooth enamel dissolution (decalcification) inhibitor of the present invention has an excellent inhibitory effect on the dissolution of tooth enamel, and is therefore useful for maintaining teeth in a healthy condition and keeping the oral cavity healthy.

### BEST MODE FOR CARRYING OUT THE INVENTION

The tooth enamel dissolution inhibitor of the present invention comprises at least one member selected from the group consisting of guar gum, glucomannan, β-cyclodextrin, tragacanth gum, carrageenan, locust bean gum, hyaluronic acid, pullulan, pectin, and xanthan gum (hereinafter may be simply referred to as active ingredients).

The above active ingredients are well known components, which have actually been used in the food and pharmaceutical fields. The molecular weight of these active ingredients used in the dissolution inhibitor of the invention is not limited. Among these active ingredients, the present invention can use hyaluronic acids in the form of a salt.

Among these active ingredients, guar gum and glucomannan are preferable for the present invention, from the viewpoint of more effectively enhancing the inhibition of tooth enamel dissolution.

In the invention, these active ingredients may be used singly or in a combination of two or more.

The dissolution inhibitor of the invention exhibits a particularly excellent inhibitory activity on the dissolution of tooth enamel caused by acid, and more particularly on the dissolution of tooth enamel caused by citric acid, malic acid, or ascorbic acid. In other words, the dissolution inhibitor of the invention is desirably used simultaneously with or after the intake of acid-containing foods, particularly foods containing citric acid, malic acid, or ascorbic acid.

The dissolution inhibitor of the invention is designed to inhibit the dissolution of tooth enamel by being applied to the oral cavity, and will be used in the fields of foods, medicines, and oral cavity products.

According to the dissolution inhibitor of the invention, the above-described active ingredients can be directly used as foods, medicines, or oral cavity products. In addition to the active ingredients, the dissolution inhibitor may contain a carrier that is acceptable from the standpoint of food hygiene or pharmacology, and may contain additives or other components if necessary, to be used as foods, medicines (including quasi-medical drugs), or oral cavity products.

The usage of the dissolution inhibitor of the present invention is not limited. For example, the dissolution inhibitor may be applied to the oral cavity in 1 to 10 divided doses per day and each dose may contain 0.001 to 20 g of active ingredients. The dissolution inhibitor is, when used, desirably held in the oral cavity for, e.g., about one second or more after application to the oral cavity.

When the dissolution inhibitor of the present invention is prepared as a food, the inhibitor may suitably contain, in addition to the above active ingredient(s), if necessary, sweeteners, food colors, preservatives, thickening agents, stabilizers, gelling agents, starch adhesives, antioxidants, couplers, decolorants, fungicides (anti-mold agents), yeast food, gum bases, flavoring agents, acidulants, seasonings, tofu coagulants, emulsifiers, pH adjustors, brines, leavens, nutrient supplements, and the like. When the dissolution inhibitor of the present invention is prepared as a food, the inhibitor may take the form of, e.g., beverages (carbonated beverages, soft drinks, milk beverages, alcoholic beverages, fruit-juice-containing beverages, teas, energy drinks, etc.), powdered beverages (powdered juices, powdered soups, etc.), snacks (gum, tablets, candies, cookies, gummy candies, rice crackers, biscuits, jelly, etc.), breads, noodles, cereals, jams, seasonings (sauce, dressing, etc.), and the like. Among these, soft drinks, fruit-juice-containing beverages, gum, tablets, and gummy candies may be preferable. Moreover, the dissolution inhibitor prepared as a food can be used as a food for inhibiting the dissolution of tooth enamel. For example, it is useful as a food for specified health foods, dietary supplements, a food for the sick, etc. When the dissolution inhibitor of the present invention is prepared as a food, the proportion of the above-mentioned active ingredient(s) in the food may be suitably determined depending on the type of active ingredient(s) to be used, the form of the food, the dose of the active ingredient (s) per day, etc. For example, the proportion of the total weight of the active ingredients is 0.001 to 100 wt.%, preferably 0.001 to 80 wt.%, and more preferably 0.005 to 50 wt.%, of the total weight of the food.

When the dissolution inhibitor of the present invention is prepared as a medicine, the inhibitor may arbitrarily contain, in addition to the above active ingredient(s), if necessary, binders, disintegrators, lubricants, humectants, buffers, preservatives, flavoring agents, etc. When the inhibitor is prepared as a medicine, the form is not limited. For example, the inhibitor may take the form of films, lozenges, solutions, powders, tablets, granules, capsules, syrups, etc. Among these, solutions, films, and lozenges are preferable. When the dissolution inhibitor of the present invention is prepared as a medicine, the proportion of the above-mentioned active ingredient(s) in the medicine may be suitably selected depending on the type of active ingredient(s) to be used, the form of the medicine, the dose of active ingredient(s) per day, etc. For example, the proportion of the total weight of the active ingredient(s) is 0.001 to 100 wt.%, preferably 0.001 to 80 wt.%, and more preferably 0.005 to 50 wt.%, of the total weight of the medicine. The dissolution inhibitor prepared as a medicine is used as a medicine for inhibiting the dissolution of tooth enamel.

When the dissolution inhibitor of the present invention is prepared as an oral cavity product, the inhibitor may contain, if necessary, various kinds of additives, such as surfactants, coloring agents (dyes, pigments), flavoring agents, preservatives, germicides (antimicrobial agents), thickeners, antioxidants, sequestering agents, refreshing agents, deodorants, and pH adjustors. The oral cavity product may specifically take the form of, e.g., dentifrice, mouthwashes, breath fresheners, gargles, etc. When the dissolution inhibitor of the present invention is prepared as an oral cavity product, the proportion of the above-mentioned active ingredients in the oral cavity product may be suitably selected depending on the type of active ingredient(s) to be used, the form of the oral cavity agent, and the dose of the active ingredient(s) per day, etc. For example, the proportion of the total weight of the active ingredient(s) is 0.001 to 100 wt.%, preferably 0.001 to 80 wt.%, and more preferably 0.005 to 50 wt.%, of the total weight of the oral cavity product. The dissolution inhibitor prepared as an oral cavity product is used as an oral cavity product for inhibiting the dissolution of tooth enamel.

### Examples

The present invention is described below with reference to Test Examples and Examples, which, however, do not limit the scope of the invention.

### Test Example 1

In order to evaluate the inhibitory activity of subject substances on the dissolution (decalcification) of tooth enamel, the following tests were conducted using sintered hydroxyapatite as a tooth enamel model.

Each subject substance with a prescribed concentration, as shown in Table 1, was added to 2 wt.% citric acid prepared with ultrapure water, and the pH of the mixture was adjusted to 2.5 with sodium hydrate. Thus, 10 ml of test solution was prepared. The test solution (10 ml) and a piece (720 mg, 10 x 10 x 2 mm) of sintered hydroxyapatite (APP-100; PENTAX Corp.) were put into a 15-ml polypropylene tube and the tube was sealed. The tube was shaken horizontally at about 120 times per minute for two days. After the shaking, the amount of phosphorus dissolved from the sintered hydroxyapatite was measured in each test solution by high-frequency inductively coupled plasma atomic emission spectrophotometry (ICP-AES). Another test was conducted as a negative control in the same manner as above, using a 2 wt.% citric acid solution (adjusted to pH 2.5 with sodium hydrate) as a test solution. Still another test was conducted as a positive control in the same manner as above, using CaCl₂ (10 mM) as a subject substance. Taking the amount of phosphorus dissolved in the negative control as 100%, the phosphorus dissolution rate (%) was calculated for each subject substance.

Table 1 shows the results. The results revealed that guar gum, glucomannan, β-cyclodextrin, tragacanth gum, carrageenan, locust bean gum, hyaluronic acid, pullulan, pectin, and xanthan gum effectively suppressed phosphorus dissolution from the sintered hydroxyapatite due to citric acid. Particularly, guar gum and glucomannan were confirmed to exhibit an extremely high inhibitory effect on phosphorus dissolution from the sintered hydroxyapatite.

**Table 1**

| Subject Substance | Concentration | Phosphorus Dissolution Rate (%) |
|---|---|---|
| Negative Control | - | 100 |
| CaCl₂ | 10 mM | 75.0 |
| Pectin | 0.5 wt.% | 69.6 |
| Guar gum. | 0.05 wt.% 0.2 wt.% | 89.8 43.5 |
| Glucomannan | 0.05 wt.% 0.2 wt.% | 92.7 40.6 |
| β-cyclodextrin | 0.2 wt.% 0.5 wt.% | 98.6 79.7 |
| Tragacanth gum | 0.2 wt.% 0.5 wt.% | 94.2 29.0 |
| Carrageenan | 0.2 wt.% 0.5 wt.% | 88.4 75.4 |
| Locust bean gum | 0.2 wt.% 0.5 wt.% | 79.7 58.0 |
| Hyaluronic acid | 0.2 wt.% 0.5 wt.% | 78.3 52.2 |
| Pullulan | 0.2 wt.% 0.5 wt.% | 78.3 78.3 |
| Xanthan gum | 0.05 wt.% 0.2 wt.% 0.5 wt.% | 86.9 68.1 23.2 |
| Propylene glycol ester alginate | 0.2 wt.% 0.5 wt.% | 107.2 98.6 |
| Hydroxypropylcellulose | 0.2 wt.% 0.5 wt.% | 97.1 95.7 |
| Gum arabic | 0.2 wt.% 0.5 wt.% | 88.4 91.3 |

Example 1 Gum

| | (Wt.%) |
|---|---|
| Gum base | 20 |
| Sorbitol | 40 |
| Maltitol | 5 |
| Mannitol | 20 |
| Malic acid | 10 |
| Guar gum | 1 |
| Glycerol | Suitable amount |
| Flavoring agent | Suitable amount |
| Total | 100 |

### Example 2 Gum

In the composition of Example 1, guar gum was replaced by the same amount of glucomannan, β-cyclodextrin, tragacanth gum, carrageenan, locust bean gum, hyaluronic acid, pullulan, pectin, or xanthan gum.

### Example 3 Mouthwash

| | (Wt.%) |
|---|---|
| Ethanol | 15 |
| Glycerol | 10 |
| Polyoxyethylene hydrogenated castor oil | 2 |
| Sodium saccharin | 0.15 |
| Sodium benzoate | 0.05 |
| Citric acid | 10 |
| Pectin | 1 |
| Flavoring agent | Suitable amount |
| Colorant | Suitable amount |
| Purified water | Suitable amount |
| Total | 100% |

### Example 4 Mouthwash

In the composition of Example 3, pectin was replaced by the same amount of guar gum, glucomannan, β-cyclodextrin, tragacanth gum, carrageenan, locust bean gum, hyaluronic acid, pullulan, or xanthan gum.

### Example 5 Breath Freshener

| | (Wt.%) |
|---|---|
| Glucomannan | 2 |
| L-menthol | 1 |
| Sodium L-ascorbate | 20 |
| Sucrose | 76 |
| Flavoring agent | Suitable amount |
| Total | 100% |

### Examples 6 Breath Freshener

In the composition of Example 5, glucomannan was replaced by the same amount of guar gum, β-cyclodextrin, tragacanth gum, carrageenan, locust bean gum, hyaluronic acid, pullulan, pectin, or xanthan gum.

## Claims

1. A tooth enamel dissolution inhibitor comprising at least one member selected from the group consisting of guar gum, glucomannan, β-cyclodextrin, tragacanth gum, carrageenan, locust bean gum, hyaluronic acid, pullulan, pectin, and xanthan gum.

2. The dissolution inhibitor according to claim 1 comprising guar gum and/or glucomannan.

3. The dissolution inhibitor according to claim 1, which is used for inhibiting tooth enamel dissolution caused by acid.

4. A food for inhibiting tooth enamel dissolution comprising at least one member selected from the group consisting of guar gum, glucomannan, β-cyclodextrin, tragacanth gum, carrageenan, locust bean gum, hyaluronic acid, pullulan, pectin, and xanthan gum.

5. The food for inhibiting tooth enamel dissolution according to claim 4 comprising guar gum and/or glucomannan.

6. A medicine for inhibiting tooth enamel dissolution comprising at least one member selected from the group consisting of guar gum, glucomannan, β-cyclodextrin, tragacanth gum, carrageenan, locust bean gum, hyaluronic acid, pullulan, pectin, and xanthan gum.

7. The medicine for inhibiting tooth enamel dissolution according to claim 6 comprising guar gum and/or glucomannan.

8. The medicine for inhibiting tooth enamel dissolution according to claim 6, which is used for inhibiting tooth enamel dissolution caused by acid.

9. An oral cavity product for inhibiting tooth enamel dissolution comprising at least one member selected from the group consisting of guar gum, glucomannan, β-cyclodextrin, tragacanth gum, carrageenan, locust bean gum, hyaluronic acid, pullulan, pectin, and xanthan gum.

10. The oral cavity product for inhibiting tooth enamel dissolution according to claim 9 comprising guar gum and/or glucomannan.

11. The oral cavity product for inhibiting tooth enamel dissolution according to claim 9, which is used for inhibiting tooth enamel dissolution caused by acid.

12. A method for inhibiting tooth enamel dissolution comprising applying an effective amount of at least one member selected from the group consisting of guar gum, glucomannan, β-cyclodextrin, tragacanth gum, carrageenan, locust bean gum, hyaluronic acid, pullulan, pectin, and xanthan gum, to an oral cavity.

13. The method according to claim 12 applying an effective amount of guar gum and/or glucomannan to an oral cavity.

14. The method according to claim 12, which is a method for inhibiting tooth enamel dissolution caused by acid.

15. Use of at least one member selected from the group consisting of guar gum, glucomannan, β-cyclodextrin, tragacanth gum, carrageenan, locust bean gum, hyaluronic acid, pullulan, pectin, and xanthan gum, for production of a tooth enamel dissolution inhibitor.

16. Use of guar gum and/or glucomannan for production of a tooth enamel dissolution inhibitor.

17. The use according to claim 15, wherein the tooth enamel dissolution inhibitor is used for inhibiting tooth enamel dissolution caused by acid.
